# EUROPEAN PATENT APPLICATION

(11) **EP 2 236 492 A1**
(43) Date of publication of application: **06.10.2010**
(21) Application number: 08858498.2
(22) Date of filing: 27.11.2008
(51) Int. Cl.: C07C 237/22, A61K 31/197

(54) **NOVEL DICARBOXYLIC AMINO ACID DERIVATIVES AND THE USE THEREOF IN THE TREATMENT OF NEURODEGENERATIVE DISEASES**

(30) Priority: 10.12.2007 ES 200703264
(71) Applicant: Consejo Superior De Investigaciones Científicas, 28006 Madrid (ES); Universidad Autónoma de Madrid, 28049 Cantoblanco Madrid (ES); Conde Ruzafa, Santiago, 28006 Madrid (ES)
(72) Inventor: CONDE RUZAFA, Santiago, E-28006 Madrid (ES); RODRIGUEZ FRANCO, Maria Isabel, E-28006 Madrid (ES); ARCE GARCIA, Mariana Paula, E-28006 Madrid (ES); GONZALEZ MUÑOZ, Gema Cristina, E-28006 Madrid (ES); VILLARROYA SÁNCHEZ, Mercedes, E-28049 Cantoblanco (madrid) (ES); GARCÍA LÓPEZ, Manuela, E-28049 Cantoblanco (Madrid) (ES); GARCIA GARCIA, Antonio, E-28049 Cantoblanco (madrid) (ES)
(74) Representative: Pons Ariño, Angel
(86) International application number: PCT/ES2008/070221
(87) International publication number: WO 2009/074706

(57) **Abstract**

The invention relates to products of general formula (I) and the use thereof for producing useful medicaments for treating neurodegenerative diseases such as Alzheimer's disease or, more generally, any disease or pathology caused by the alteration of the biological functions on which said products act.

## Description

### FIELD OF THE INVENTION

The present invention is included in the field of research and pharmaceutical industry. Particularly it focuses on the synthesis of new derivatives of dicarboxylic amino acids and their application in the treatment of neurodegenerative diseases (ND).

### BACKGROUND OF THE INVENTION

The term dementia describes a chronic o progressive alteration of cortical or sub-cortical functions of the brain, with the result of more or less complex cognitive failures, usually accompanied by perturbations of the general state, behavior and personality. Many of the variations in brain functions are pathological processes known as ND, whose main common characteristic is the loss of neurons in certain areas of the central nervous system (CNS) which vary with the ND and are associated to age as a factor of risk. In the last years, as a consequence of the improvement in the sanitary and health conditions, the life expectation has increased in a spectacular manner (Eurostat Pocketbooks. Living conditions in Europe. Data 2002-2005. 2007 edition.), bringing with it the undesirable consequence of an increase in the ND and senile dementias. Among them, Alzheimer's disease (AD) is the most common ND, responsible of approximately two thirds of the total cases of dementia (varying between 42% and 81% depending on different studies), with a prevalence highly related to age, which is close to 50% in the population older than 85 (N. Engl. J. Med. 2003, 348, 1356-1364).

Until today, in spite of the great amount of effort and money invested, the mechanism or mechanisms that trigger the different processes (progressive disappearance of neuronal tissue, amyloid beta peptide aggregation, tau protein hyperphosphorylation, oxidative and inflammatory processes, mutation in certain genes, etc.) which are present in the AD pathology are still unknown, although there exist various hypothesis which relate some of these processes among them. However, it seems clear that AD is the consequence of the combination of more than one of these pathological processes, and that the aberrant formation of aggregates of amyloid peptide (oligomers and protofibrils to give place to the amyloid plaques) together with uncontrolled oxidative processes are in the origin of the AD pathology (Pharmacol. Sci. 1991, 12, 383-388).

Moreover, in AD and other ND a number of cognitive failures due to a decrease in the levels of neurotransmitters involved in the synaptic transmission are present. In the case of AD the failure in the cholinergic neurotransmission is especially significant, with levels of acetylcholine (ACh) highly diminished (Clin. Neuropharmacol. 2004, 27, 141-149).

It is also known that acetylcholinesterase (AChE) exerts a double biological function since, besides being responsible of the hydrolysis of ACh in its catalytic site, it favors the aggregation of the amyloid peptide to give neurotoxic species by means of the activity centered in an anionic peripheral site located in the entrance of the gorge conducting to the catalytic site (FEBS Lett. 2005, 579, 5260-5264).

Considering this situation, and knowing that the massive loss of neurons is practically irreversible, it seems obvious the convenience of having medicines able to act in the initial phases of the illness or, even better, of producing a neuroprotective activity able to stop the advance of the illness when the first symptoms appear or even before, in an ideal case, if an adequate early diagnostic system could be available. Also, taking into account the different processes participating in the pathology of AD, it would be highly desirable that these compounds were able to act over more than one therapeutic target like, for example, to inhibit AChE with the consequent improvement in the cholinergic neurotransmission and therefore, relieve in the cognitive failures, to inhibit the aggregation of amyloid peptide which would slow the progress of the illness, or to reduce the so-called oxidative stress which is produced as a consequence of the loss of balance between the oxidative species generated by the brain metabolism and the endogenous antioxidant protective mechanisms. These are the medicines or drugs named "of multifunctional action".

### DESCRIPTION OF THE INVENTION

### Brief description

The object of the present invention is referred to new dicarboxylic amino acid derivatives. These compounds have not yet been described and exhibit one or more than one biological activities in a single molecule, which made them as potentially useful compounds in the treatment of ND in general and AD in particular.

### Detailed description

The present invention is based on the synthesis of a family of new compounds of general formula (I), not yet described. These compounds were pharmacologically evaluated and showed interesting biological activities:
- Antioxidation and neuroprotection
- Inhibition of AChE
- Displacement of propidium iodide from the peripheral anionic site of the enzyme acetylcholinesterase.
- Penetration into the CNS.

Thus, these products are potentially useful as therapeutic agents. The results obtained indicate that they are a family of compounds displaying a multifunctional biological action.

So, in a first aspect, the present invention provides compounds of general formula (I): 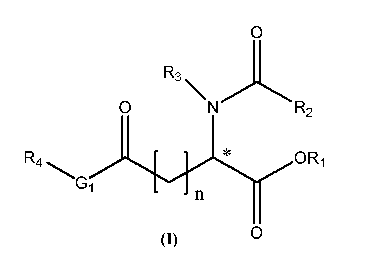 wherein:
- R₁: represents a hydrogen atom or an aryl, heteroaryl, alkyl, haloalkyl, aminoalkyl, ammoniumalkyl group and, in general, any straight or branched chain alkyl group which may optionally be substituted by any chemical group.
- R₂, R₃: represent a hydrogen atom or an aryl, heteroaryl, straight or branched chain alkyl groups which may optionally be substituted by any chemical group, carbamates where R₂ represents -O-aryl or straight or branched chain -O-alkyl groups which may optionally be substituted by any chemical group, urea derivatives where R₂ represents -NH-aryl or straight or branched chain -N- alkyl groups which may optionally be substituted by any chemical group, imides, etc. In general, any chemical group capable of bringing on the molecule represented by formula (I).
- n: has the values 1 or 2.
- *: points out the amino acid chiral carbon, and may be the R enantiomer, S enantiomer or a mixture of both enantiomers at any ratio.
- G₁: represents O, mono or disubstituted N, CH₂, S or, in general, any group or chemical structure which could be used as a link between the amino acid moiety and R₄.
- R₄: represents any chemical structure formed by any combination of unsubstituted or substituted alkyl, aryl or heteroaryl groups.
or any isomer, pharmaceutically acceptable salt and/or solvate of it.

In a second aspect, the invention provides the use of a compound of formula (I) as a medicament, in particular in the manufacture of a medicament with neuroprotective activity potentially useful in the treatment of ND or, in general, pathologies which could be treated with compounds displaying biological activities like those exhibited by the compounds described in the present invention, or any of their therapeutically acceptable salts, derivatives, pro-drugs or solvates.

The compounds referred to in this invention can be in crystalline forms as free compounds or as solvates and it is intended that both forms are within the scope of the present invention. In this sense, the term "solvate" as used herein, includes both pharmaceutically acceptable solvates, or solvates of the compound of formula (I) that can be used in the preparation of a medicament, and non-pharmaceutically acceptable solvates, which can be useful in preparing pharmaceutically acceptable salts and solvates. The nature of the pharmaceutically acceptable solvate is not critical, provided it is pharmaceutically acceptable. In a particular embodiment, the solvate is a hydrate. The solvates may be obtained by conventional methods of solvation which are well known by the experts in the field.

For its application in therapy, compounds of formula (I) including its isomers, salts, pro-drugs or solvates, will be, preferably, in a pharmaceutically acceptable or substantially pure form, that is, in a pharmaceutically acceptable level of purity excluding normal pharmaceutical additives such as diluents and carriers, and not including any material considered as toxic at normal dosage levels. The purity levels for the active ingredient are preferably above 50%, more preferably above 70%, yet more preferably above 90%. In an even more preferred embodiment, the purity of the compound of formula (I) is over 95%, or its salts, solvates or pro-drugs.

Unless otherwise indicated, the compounds of the invention also include compounds that differ only in the presence of one or more isotopically enriched atoms. For example, compounds having this structure, except for the replacement of a hydrogen by a deuterium or tritium, or the replacement of a carbon atom by a carbon enriched in ¹³C or ¹⁴C or ¹⁵N enriched nitrogen are within the scope of this invention.

The compounds described in this invention, their pharmaceutically acceptable salts, pro-drugs and / or solvates and pharmaceutical compositions containing them can be used coupled with other additional drugs to provide a combining therapy. These additional drugs may be part of the same pharmaceutical composition or, alternatively, can be provided in the form of a separate composition for administration simultaneously or not to the pharmaceutical composition comprising a compound of formula (I), a pro-drug, solvate, derivative or a pharmaceutically acceptable salt thereof.

The compounds of formula (I) described at the present invention may be obtained or produced by a chemical synthetic route or obtained from a natural material of different origins. In another aspect, the present invention describes a method for obtaining the compounds of formula (I) or an isomer, pharmaceutically acceptable salt and / or solvate of them characterized by the following steps:
a) Reaction of a product of formula (II) 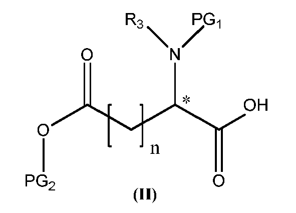 in which the PG₁ represents a protecting group of amino groups and PG₂ is a protecting group of carboxyl groups, and an alcohol molecule of formula R₁-OH in an anhydrous solvent to obtain compounds of formula (III). 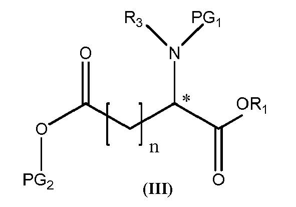
b) Deprotection of the carboxylic acid group in the compounds of formula (III) by cleaving the hydroxyl protecting group PG₂ to yield the compound of formula (IV). 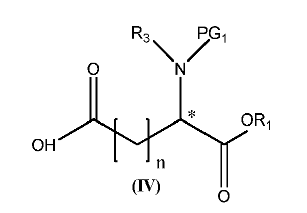
c) Reaction of the compound of formula (IV) with a compound of formula H-G₁-R₄ in the presence of a coupling agent to give compounds of formula (Ia). 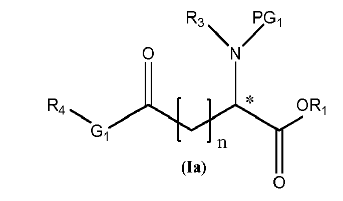
Optionally, when R₂ in the compound of formula (Ia) is a *tert-*butyloxyl group, the compound is hydrolysed by treatment with strong acid. The resulting product is subsequently acylated with an acyl chloride of formula R₂COCl or an anhydride of formula (R₂CO)₂O in an anhydrous non-polar solvent.

In a particular implementation of the present invention in the compounds of formula (I), R₁ is a C₂-C₈ straight or branched alkyl group, preferably a C₅-C₇ straight chain alkyl group and, more preferably, an *n*-hexyl group.

In another implementation of the present invention in the compounds of formula (I), the R₃ group is a hydrogen atom or a C₁-C₃ alkyl group, more preferably a hydrogen atom.

In another implementation of the present invention in the compounds of formula (I), the G₁ group is an amino group optionally substituted by one or two C₁-C₃ alkyl groups, more preferably an -NH- group.

In still another implementation of the present invention in the compounds of formula (I), n has the preferred value of 2.

It is also an implementation of this invention when R₄ represents a group piperidine-4-yl-ethyl optionally substituted in the compounds of formula (I), preferably a group piperidine-4-yl-ethyl which may be substituted in its nitrogen atom with an arylalkyl group, preferably a benzyl group optionally substituted with 1-3 groups selected from C₁-C₃ alkyl, C₁-C₃ alkoxy, cyano, nitro and halogen, more preferably an unsubstituted benzyl group.

The following compounds are examples of compounds which are according to this invention:
- *n*-Hexyl 2-benzyloxycarbonylamino-4-[2-(1-benzylpiperidin-4-yl)-ethylcarbamoyl]-butyrate.
- *n*-Hexyl 4-[2-(1-benzylpiperidin-4-yl)-ethylcarbamoyl]-2*-tert-*butoxycarbonylamino-butyrate.
- *n*-Hexyl 2-benzoylamino-4-[2-(1-benzylpiperidin-4-yl)-ethylcarbamoyl]-butyrate.
- *n-*Hexyl 2-[(benzo[b]tiofen-2-carbonyl)-amino]-4-[2-(1-benzyl-piperidin-4-yl)-ethylcarbamoyl]-butyrate.
- *n-*Hexyl 4-[2-(1-benzyl-piperidin-4-yl)-ethylcarbamoyl]-2-[2-(6-chlorobenzo[*b*]thiophen-2-yl)-acetylamino]-butyrate.
- *n*-Hexyl 4-[2-(1-benzyl-piperidin-4-yl)-ethylcarbamoyl]-2-[(thieno[*2,3-b*]piridin-2-carbonyl)-amino]-butyrate.
- *n*-Hexyl 4-[2-(1-benzyl-piperidin-4-yl)-ethylcarbamoyl]-2-[(thieno[*2,3-b*]thiophen-2-carbonyl)-amino]-butyrate.
- *n*-Hexyl 4-[2-(1-benzyl-piperidin-4-yl)-ethylcarbamoyl]-2-[(thiophen-2-carbonyl)-amino]-butyrate.
- *n*-Hexyl 4-[2-(1-benzyl-piperidin-4-yl)-ethylcarbamoyl]-2-[(4-bromo-thiophen-2-carbonyl)-amino]-butyrate.
all of the names of the groups are common in organic chemistry

An additional object of this invention is a pharmaceutical composition, useful for treating ND or, in general, diseases which could benefit from the biological activities displayed by the products described in this invention, hereinafter pharmaceutical composition, which comprises a compound in therapeutically effective amount of formula (I), or mixtures thereof, a salt, pro-drug, pharmaceutically acceptable solvate or stereoisomer thereof together with a carrier, adjuvant or pharmaceutically acceptable vehicle for the administration to a patient.

Another particular object of the invention is the use of the pharmaceutical composition of the invention for the treatment or prevention of ND such as AD, Creutzfeldt-Jakob disease, Parkinson's or Huntington, dementia with Lewy bodies or, in general, any disease which could benefit from the biological activities displayed by the products described in this invention.

Still another particular object of the invention is a pharmaceutical composition in which the compound of formula (I) is a compound or mixture of compounds belonging, for illustrative purposes and without limiting the scope of the invention, to the following group:
- *n*-Hexyl 2-benzyloxycarbonylamino-4-[2-(1-benzylpiperidin-4-yl)-ethylcarbamoyl]-butyrate.
- *n*-Hexyl 4-[2-(1-benzylpiperidin-4-yl)-ethylcarbamoyl]-2-*tert-*butoxycarbonylamino-butyrate.
- *n*-Hexyl 2-benzoylamino-4-[2-(1-benzylpiperidin-4-yl)-ethylcarbamoyl]-butyrate.
- *n*-Hexyl 2-[(benzo[*b*]tiofen-2-carbonyl)-amino]-4-[2-(1-benzyl-piperidin-4-yl)-ethylcarbamoyl]-butyrate.
- *n*-Hexyl 4-[2-(1-benzyl-piperidin-4-yl)-ethylcarbamoyl]-2-[2-(6-chloro-benzo[*b*]thiophen-2-yl)-acetylamino]-butyrate.
- *n*-Hexyl 4-[2-(1-benzyl-piperidin-4-yl)-ethylcarbamoyl]-2-[(thieno[2,3-*b*]piridin-2-carbonyl)-amino]-butyrate.
- *n*-Hexyl 4-[2-(1-benzyl-piperidin-4-yl)-ethylcarbamoyl]-2-[(thieno[2,3-*b*]thiophen-2-carbonyl)-amino]-butyrate.
- *n*-Hexyl 4-[2-(1-benzyl-piperidin-4-yl)-ethylcarbamoyl]-2-[(thiophen-2-carbonyl)-amino]-butyrate.
- *n*-Hexyl 4-[2-(1-benzyl-piperidin-4-yl)-ethylcarbamoyl]-2-[(4-bromo-thiophen-2-carbonyl)-amino]-butyrate.

The adjuvants and vehicles that can be used in these compositions are pharmaceutically acceptable adjuvants and vehicles known to those skilled in the art and commonly used in the preparation of therapeutic compositions.

In the sense used in this description, the term "therapeutically effective amount" or dose refers to the amount of agent or compound capable of developing the therapeutic action determined by their pharmacological properties. It is calculated to produce the desired effect and generally will be determined, among other things, by combining the characteristics of compounds and patients, including age, state of the patient, severity of the disturbance or disorder, and route and frequency of the administration.

In another particular embodiment, the therapeutic composition may be prepared in a solid form or as an aqueous suspension in a pharmaceutically acceptable diluent. The therapeutic composition provided by this invention may be administered by any appropriate route of administration, for which the composition is formulated in the suitable pharmaceutical form to the chosen route of administration. In a particular embodiment, the administration of the therapeutic composition provided by this invention may be effected by oral, topical, rectal or parenteral (including subcutaneous, intraperitoneal, intradermal, intramuscular, intravenous, etc.). A review of the different pharmaceutical forms of administration of drugs and the excipient needed for obtaining them can be found, for example, in the "Treaty of Galenic Pharmacy, C. Faulí i Trillo, 1993, Luzán 5, SA Ediciones, Madrid, or in other common or similar to the Spanish and United States Pharmacopoeias.

The employment of the compounds of the invention is compatible with protocols in which the compounds of formula (I) or their mixtures are used by themselves or in combination with other treatments or any medical procedure.

In another aspect, this patent discloses a method for the treatment of patients affected by ND, consisting of the administration of therapeutically effective amounts of a compound of formula (I) or a pharmaceutical composition including it, to individuals affected by these diseases. Examples of ND covered in this invention are AD, Creutzfeldt-Jakob disease, Parkinson's or Huntington, dementia with Lewy bodies or, in general, any disease which could benefit from the biological activities shown by the products described in the present invention.

### PREPARATION PROCEDURES

According to the present invention, the compounds of formula (I) wherein R₁, R₂, R₃, R₄, G₁, and n have the meanings defined in claim 1, can be prepared as described below, from commercially available products or from products whose preparation is sufficiently described in the state of the art.

In this report the following abbreviations are used with the meanings shown below:
Cbz refers to a benzyloxycarbonyl group, also called carbobenzoxy,
Boc refers to *tert*-butyloxycarbonyl group,
tBu represents *tert*-butyl group,
Bn represents benzyl group,
L and D indicate the configuration of the enantiomer,
PyBOP refers to benzotriazol-1-yl-oxytripyrrolidinophosphonium hexafluorophosphate,
EDC refers to *N*-ethyl-*N*'(3-dimethylaminopropyl) carbodiimide,
HOBt refers to *N*-hydroxybenzotriazole,
DMAP refers to dimethylaminopyridine,
DMF refers to dimethylformamide, and
TFA is trifluoroacetic acid

All groups, reagents and solvents are common in organic synthesis, especially in the field of synthesis of amino acids.

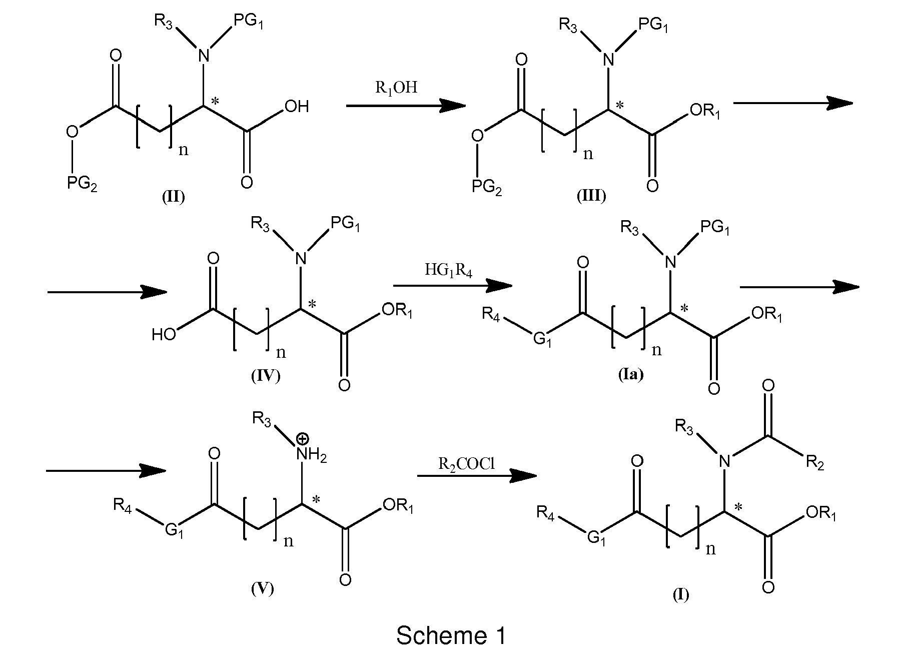

In a first stage, the dicarboxylic amino acids of formula (II), wherein PG₁ represents a protective group of hydroxyl groups such as benzyloxycarbonyl (Cbz) and *tert*-butyloxycarbonyl (Boc) and PG₂ is a protecting group of amino groups as *tert*-butyl (t-Bu) or benzyl (Bn), are esterified by reaction with an alcohol of formula R₁-OH in an anhydrous solvent such as methylene chloride, in the presence of a base such as triethylamine and a catalyst such as PyBOP, giving compounds of formula (III). The reaction is performed under an inert atmosphere and preferably at 0 °C during the time required by the chemical transformation, usually between 15 and 20 hours, as showed by the thin layer chromatography controls. Once the reaction is finished, the solvent is evaporated and the residue is purified by flash column chromatography on silica gel using mixtures of hexane / ethyl acetate as eluent.

Then, the protecting group of hydroxyl groups (PG₂) is removed, following procedures that are known to an expert in the art, obtaining the products of formula (IV). For example, when the protecting group is a benzyloxycarbonyl group the compounds of formula (III) are treated with a strong acid such as trifluoroacetic acid in the presence of a solvent such as methylene chloride. When the protecting group is *tert*-butyloxycarbonyl group, the compounds of formula (III) are subjected to catalytic hydrogenation, eg with palladium on activated carbon.

Then, compounds of formula (IV) are condensed with a compound of formula H-G₁-R₄ in the presence of a coupling agent such as EDC, HOB, triethylamine, and catalytic amounts of DMAP to yield compounds of formula (Ia). Reaction is performed at room temperature with agitation in anhydrous dimethylformamide solution and its course is followed by thin layer chromatography. Once the reaction is completed, solvent is evaporated and the product is purified by flash column chromatography on silica gel, for example using a mixture of methylene chloride: methanol (95:5) as eluent. This last step may not be necessary in case of continuing the synthesis procedure as described in the following paragraph.

The compounds of formula (Ia) are intermediates for obtaining compounds of formula (I) and are also the final compounds of formula (I) in which R₂ is a benzyloxy or a *tert*-butyloxy group.

To obtain a compound of formula (I) in which the group R₂ is different from benzyloxy or *tert*-butyloxy group, the next synthetic step is the hydrolysis of compound (Ia) in which R₂ is a *tert*-butyloxycarbonyl group. Treatment of (Ia) with a strong acid such as trifluoroacetic acid in the presence of a solvent such as methylene chloride yields a compound of formula (V).

Finally, compounds of formula (V) are acylated by treatment with an acyl chloride of formula R₂COCl or an anhydride of formula (R₂CO)₂O in a non-polar solvent such as anhydrous methylene chloride, in the presence of at least two equivalents of a base such as triethylamine, cooling and shaking.

### EXAMPLES

### Example 1. Synthesis of compounds of the invention

**Example 1.1. Synthesis of *n*-hexyl 2-benzyloxycarbonylamino-4-[2-(1-benzylpiperidin-4-yl)-ethylcarbamoyl]-butyrate.** This compound was synthesized as described in the previous section (Preparation Procedures), following the synthesis shown in Scheme 1. Colorless solid of melting point: 88-90 ºC. MS (ESI⁺): *m*/*z* = 566 [M+H]⁺. ¹H-NMR (300 MHz, MeOD) δ: 7.43 (s, 10H, H_{3"}-H_{7"}, H_{10'}-H_{14'}), 5.10 (s, 2H, H_{1"}) 4.50 (m, 1H, H_{α}), 4.14 (m, 2H, H₁), 4.13 (s, 2H, H_{8'}), 3.34-3.29 (m, 3H, H_{6'eq}, H_{5'eq}, H_{3'}), 3.16 (m, 2H, H_{1'}), 2.74 (m, 2H, H_{6'ax}, H_{5'ax}), 2.35 (m, 2H, H_{γ}), 2.28 (m, 1H, H_{β}), 2.06 (m, 1H, H_{β}) 1.87 (m, 2H, H_{7'eq}, H_{4'eq}), 1.64 (m, 2H, H₂), 1.42 (m, 2H, H_{2'}), 1.34-1.26 (m, 8H, H_{7'ax}, H_{4'ax}, H₃, H₄, H₅), 0.86 (t, 3H, *J* = 7.0 Hz, H₆) ppm. ¹³C-NMR (75 MHz, MeOD) δ: 174.6 (*C*OC_{γ}), 173.3 (COC_{α}), 164.5 (*C*ONHC_{α}), 139.6 (C_{2"}, C_{9'}), 132.2 (C_{3"}, C_{7"}), 132.0 (C_{10'}, C_{14'}), 130.5 (C_{5"}), 130.2 (C_{12'}), 130.1 (C_{11'}, C_{13'}), 128.9 (C_{4"}, C_{6"}), 66.5 (C_{1"}), 65.3 (C₁), 62.2 (C_{8'}), 54.0 (C_{α}, C_{3'}), 53.7 (C_{5'},C_{6'}), 37.4 (C_{1'}), 36.2 (C_{2'}), 33.2 (C_{γ}), 32.5 (C_{4'}, C_{7'}), 30.7 (C₃), 29.6 (C₂), 27.8 (Cβ), 26.7 (C₄), 23.6 (C₅), 14.3 (C₆) ppm. Purity: 97% (by HPLC).

**Example 1.2. Synthesis of *n*-hexyl 4-[2-(1-benzylpiperidin-4-yl)-ethylcarbamoyl]-2-*tert*-butoxycarbonylamino-butyrate.** This compound, like the following examples, was synthesized as described in the previous section (Preparation Procedures), following the synthesis shown in Scheme 1. Colorless oil. MS (ESI+): *m*/*z* = 532 [M+H]⁺. ¹H-NMR (400 MHz, CDCl₃) δ: 7.40 (m, 5H, H_{10'}-H_{14'}), 6.46 (m, 1H, NHCOC_{γ}), 4.18-4.07 (m, 3H, H_{α}, H₁), 5.37 (m, 1H, NHC_{α}), 3.37 (s, 2H, H_{8'}), 3.22 (m, 2H, H_{1'}), 2.85 (m, 2H, H_{6'eq}, H_{5'eq}), 2.40-2.22 (m, 4H, H_{β}, H_{γ}), 1.90 (m, 2H, H_{6'ax}, H_{5'ax}), 1.61-1.52 (m, 4H, H_{7'eq}, H_{4'eq}, H₂), 1.40 (s, 9H, Boc), 1.34-1.27 (m, 11H, H_{7'ax}, H_{4'ax}, H_{2'}, H_{3'}, H₃, H₄, H₅), 0.87 (t, 3H, *J* = 6.8 Hz, H₆) ppm. ¹³C-NMR (100 MHz, CDCl₃) δ: 172.6 (COC_{γ}), 172.3 (COC_{α}), 155.9 (CONHC_{α}), 131.8 (C_{9'}), 130.8 (C_{14'}, C_{10'}), 129.8 (C_{13'}, C_{11'}), 129.2 (C_{12'}), 80.0 (C-Boc), 65.7 (C₁), 61.3 (C_{8'}), 53.1 (C_{5'},C_{6'}), 52.6 (C_{α}), 37.5 (C_{1'}), 36.6 (C_{2'}), 34.7 (C_{3'}), 32.2 (C_{γ}), 31.3 (C_{4'}, C_{7'}), 30.7 (C₃), 28.6 (C_{β}), 28.4 (C₂), 28.2 (3CH₃-Boc), 25.4 (C₄), 22.4 (C₅), 14.0 (C₆) ppm. Purity: 96% (by HPLC).

**Example 1.3. Synthesis of *n*-hexyl 2-benzoylamino-4-[2-(1-benzylpiperidin-4-yl)-ethylcarbamoyl]-butyrate.** Colorless solid of melting point: 89-91 ºC. MS (ESI⁺): *m*/*z* = 536 [M+H]⁺. ¹H-NMR (400 MHz, CDCl₃) δ: 7.85 (m, 3H, H_{3"}, H_{5"}, NHC_{α}), 7.46 (m, 3H, H_{2"}, H_{6"}, H_{4"}), 7.30 (m, 5H, H_{10'}-H_{14'}), 6.09 (m, 1H, NHCOC_{γ}), 4.69 (m, 1H, H_{α}), 4.16 (td, 2H, J = 6.33, 1.93 Hz, H₁), 3.54 (s, 2H, H_{8'}), 3.26-3.19 (m, 2H, H_{1'}), 2.90 (m, 2H, H_{6'eq}, H_{5'eq}), 2.38-2.17 (m, 4H, H_{β} H_{γ}), 2.12-1.98 (m, 2H, H_{6'ax}, H_{5'ax}), 1.66-1.60 (m, 4H, H_{7'eq}, H_{4'eq}, H₂), 1.40-1.25 (m, 11H, H_{7'ax}, H_{4'ax}, H_{2'}, H_{3'}, H₃, H₄, H₅), 0.87 (t, 3H, J = 7.02 Hz, H₆) ppm. ¹³C-NMR (100 MHz, CDCl₃) δ: 172.2 (*C*OC_{γ}), 172.0 (*C*OC_{α}), 167.4 (*C*ONHC_{α}), 133.5 (C_{1"}), 131.9 (C_{4"}), 129.5 (C_{9'}), 128.6 (C_{14'}, C_{10'}), 128.3 (C_{2"}, C_{6"}, C_{12'}), 127.3 (C_{13'}, C_{11'}), 127.1 (C_{3"}, C_{5"}), 65.9 (C₁), 63.0 (C_{8'}), 53.4 (C_{5'},C_{6'}), 52.7 (C_{α}), 37.3 (C_{1'}), 35.9 (C_{2'}), 33.1 (C_{3'}), 32.8 (C_{γ}), 31.6 (C_{4'}, C_{7'}), 31.3 (C₃), 28.5 (C₂), 28.1 (C_{β}), 25.4 (C₄), 22.5 (C₅), 14.0 (C₆). Purity: 96% (by HPLC).

**Example 1.4. Synthesis of *n*-hexyl 2-[(benzo[*b*]tiofen-2-carbonyl)-amino]-4-[2-(1-benzyl-piperidin-4-yl)-ethylcarbamoyl]-butyrate. Yellow** solid of melting point: 101-103 ºC. MS (ESI⁺): *m*/*z* = 592 [M+H]⁺. H-NMR (400 MHz, CDCl₃) δ: 7.84-7.81 (m, 2H, H_{3"}, H_{7"}), 7.76 (d, *J* = 7.1, NCH_{α}), 7.39-7.35 (m, 3H, H_{4"}, H_{5"}, H_{6"}), 7.27 (m, 5H, H_{10'}-H_{14'}), 5.96 (m, 1H, NHCOC_{γ}), 4.66-4.64 (m, 1H, H_{α}), 4.14 (m, 2H, H₁), 3.5 (s, 2H, H_{8'}), 3.25-3.17 (m, 2H, H_{1'}), 2.83 (m, 2H, H_{5'eq}, H_{6'eq}), 2.40-2.14 (m, 4H, H_{β}, H_{γ}), 1.92 (m, 2H, H_{5'ax}, H_{6'ax}), 1.64-1.53 (m, 4H, H_{4'eq}, H_{7'eq}, H₂), 1.39-1.22 (m, 11H, H_{4'ax}, H_{7'ax}, H_{2'}, H_{3'}, H₃, H₄, H₅), 0.85 (t, 3H, *J* = 6.78 Hz, H₆) ppm. ¹³C-NMR (100 MHz, CDCl₃) δ: 172.6 (*C*OC_{γ}), 171.0 (*C*OC_{α}), 162 (*C*ONHC_{α}), 141.4 (C_{2"}, C_{9"}), 139.4 (C_{8"}), 138.3 (C_{9'}), 129.6 (C_{1o'}, C_{14'}), 128.5 (C_{11'}, C_{13'}), 127.5 (C_{12'}), 126.6 (C_{4"}), 125.7 (C_{6"}), 125.4 (C_{5"}), 125.1 (C_{7"}), 123.0 (C_{3"}), 66.1 (C₁), 63.4 (C_{8'}), 53.7 (C_{α}), 53.2 (C_{5'},C_{6'}), 37.6 (C_{1'}), 36.2 (C_{2'}), 33.3 (C_{3'}), 33.0 (C_{γ}), 31.9 (C_{4'}, C_{7'}), 31.6 (C₃), 28.7 (C₂), 27.7 (C_{β}), 25.7 (C₄), 22.7 (C₅), 14.2 (C₆) ppm. Purity: 94% (by HPLC).

**Example 1.5. Synthesis of *n*-hexyl 4-[2-(1-benzyl-piperidin-4-yl)-ethylcarbamoyl]-2-[2-(6-chloro-benzo[*b*]thiophen-2-yl)-acetylamino]-butyrate.** Yellow solid of melting point: 84-86 ºC. MS (ESI⁺): *m*/*z* = 641 [M+H]⁺. ¹H-NMR (300 MHz, MeOD) δ: 7.80 (m, 3H, H_{4"}, H_{5"}, H_{7"}), 7.55 (s, 1H, H_{3"}), 7.34 (m, 5H, H_{10'}-H_{14'}), 4.39 (m, 1H, H_{α}), 4.08 (m, 2H, H₁), 3.81 (s, 2H, H_{10"}), 3.72 (s, 2H, H_{8'}), 3.18 (m, 2H, H_{1'}), 3.00 (m, 2H, H_{6'eq}, H_{5'eq}), 2.31 (m, 3H, H_{γ}, H_{β}), 2.17 (m, 2H, H_{5'ax}, H_{6'ax}), 1.93 (m, 1H, H_{β}) 1.73 (m, 2H, H_{4'eq}, H_{7'eq}), 1.54 (m, 1H, H₂), 1.39 (m, 2H, H_{2'}), 1.33-1.25 (m, 9H, H₅, H₃, H₄, H_{3'}, H_{4'ax}, H_{7'ax}), 0.84 (t, 3H, *J* = 7.0 Hz, H₆) ppm. ¹³C-NMR (75 MHz, MeOD) δ: 174.3 (*C*OC_{γ}), 173.2 (*C*OC_{α}), 172.7 (*C*ONHC_{α}), 141.5 (C_{2"}), 140.0 (C_{9"}), 135.2 (C₈"), 131.6 (C_{6"}), 131.4 (C_{9'}), 130.0 (C_{14'}, C_{10'}), 129.6 (C_{13'}, C_{11'}), 129.5 (C_{12'}), 128.0 (C_{5"}), 125.8 (C_{4"}), 125.1 (C_{7"}), 122.8 (C_{3"}), 66.4 (C₁), 63.1 (C_{8'}), 54.0 (C_{α}), 53.0 (C_{5'}, C_{6'}), 37.6 (C_{1'}), 36.3 (C_{3'}), 33.0 (C_{γ}), 32.5 (C_{2'}), 31.6 (C_{4'}, C_{7'}), 29.6 (C₅), 28.3 (C_{β}), 26.7 (C₄), 26.5 (C₃), 23.6 (C₂), 14.4 (C₆) ppm. Purity: 98% (by HPLC).

**Example 1.6. Synthesis of *n*-hexyl 4-[2-(1-benzyl-piperidin-4-yl)-ethylcarbamoyl]-2-[(thieno[*2,3-b*]piridin-2-carbonyl)-amino]-butyrate.** Yellow oil. MS (ESI⁺): *m*/*z* = 593 [M+H]⁺, 616 [M+Na]⁺. ¹H-NMR (400 MHz, CDCl₃) δ: 8.61 (dd, 1H, *J* = 4.6, 1.6 Hz, H_{6"}), 8.09 (dd, 1H, J = 8.2, 1.6 Hz, H_{4"}), 8.00 (d, 1H, *J* = 6.8 Hz, NHC_{α}), 7.78 (s, 1H, H_{3"}), 7.32-7.22 (m, 6H, H_{10'}-H_{14'}, H_{5"}), 5.88 (m, 1H, NHCOC_{γ}), 4.65 (m, 1H, CH_{α}), 4.15 (m, 2H, H₁), 3.46 (s, 2H, H_{8'}), 3.23 (m, 2H, H_{1'}), 2.81 (m, 2H, H_{5'eq}, H_{6'eq}), 2.41-2.17 (m, 4H, H_{β}, H_{γ}), 1.89 (m, 2H, H_{5'ax}, H_{6'ax}), 1.65-1.55 (m, 4H, H_{4'eq}, H_{7'eq}, H₂), 1.39-1.24 (m, 11H, H_{4'ax}, H_{7'ax}, H_{2'}, H_{3'}, H₃, H₄, H₅), 0.85 (t, 3H, *J* = 6.8 Hz, H₆) ppm. ¹³C-NMR (100 MHz, CDCl₃) δ: 172.5 (*C*OC_{γ}), 171.6 (*C*OC_{α}), 162.2 (*C*ONHC_{α}), 162.1 (C_{7"}), 148.7 (C_{6"}), 138.4 (C_{2"}), 132.7 (C_{4"}, C_{9"}), 129.3 (C_{14'}, C_{10'}), 128.2 (C_{13'}, C_{11'}), 127.1 (C_{12'}), 123.0 (C_{8"}, C_{3"}), 120.1 (C_{4"}, C_{5"}), 65.9 (C₁), 63.2 (C_{8'}), 53.5 (C_{5'},C_{6'}), 53.1 (C_{α}), 37.5 (C_{1'}), 36.0 (C_{2'}), 33.3 (C_{3'}), 32.7 (C_{γ}), 31.9 (C_{4'}, C_{7'}), 31.3 (C₃), 28.4 (C₅), 27.2 (C_{β}), 25.4 (C₄), 22.5 (C₂), 14.0 (C₆) ppm. Purity: 95% (by HPLC).

**Example 1.7. Synthesis of *n*-hexyl 4-[2-(1-benzyl-piperidin-4-yl)-ethylcarbamoyl]-2-[(thieno[*2,3-b*]thiophen-2-carbonyl)-amino]-butyrate.** Yellow solid of melting point: 100-102 ºC. MS (ESI⁺): *m*/*z* = 598 [M+H]⁺. ¹H-NMR (400 MHz, CDCl₃) δ: 7.74 (s, 1H, H_{3"}), 7.68 (d, 1H, *J* = 7.1 Hz, NHC_{α}), 7.36 (d, 1H, *J* = 5.5 Hz, H_{5"}), 7.29 (m, 5H, H_{10'}-H_{14'}), 7.23 (d, 1H, *J* = 5.5 Hz, H_{4"}), 5.99 (m, 1H, NHCOC_{γ}), 4.65 (m, 1H, H_{α}), 4.14 (m, 2H, H₁), 3.5 (s, 2H, H_{8'}), 3.23 (m, 2H, H_{1'}), 2.90 (m, 2H, H_{5'eq}, H_{6'eq}), 2.39-2.16 (m, 4H, H_{β}, H_{γ}), 2.00 (m, 2H, H_{5'ax}, H_{6'ax}), 1.65-1.59 (m, 4H, H_{4'eq}, H_{7'eq}, H₂), 1.39-1.24 (m, 11H, H_{4'ax}, H_{7'ax}, H_{2'}, H_{3'}, H₃, H₄, H₅), 0.85 (t, 3H, *J* = 7.0 Hz, H₆) ppm. ¹³C-NMR (100 MHz, CDCl₃) δ: 172.5 (*C*OC_{γ}), 171.8 (*C*OC_{α}), 162.3 (*C*ONHC_{α}), 146.3 (C_{2"}), 141.6 (C_{7"}), 141.3 (C_{9'}), 129.5 (C_{3"}), 129.0 (C_{10'}, C_{14'}), 128.3 (C_{8"}, C_{11'}, C_{13',}), 127.4 (C_{12'}), 120.9 (C_{5"}), 125.5 (C_{4"}), 65.9 (C₁), 63.0 (C_{8'}), 53.4 (C_{5'}, C_{6'}), 53.0 (C_{α}), 37.4 (C_{1'}), 36_{.}0 (C_{2'}), 33.1 (C_{3'}), 32.8 (C_{γ}), 31.5 (C_{4'}, C_{7'}), 31.3 (C₃), 28.4 (C₅), 27.5 (C_{β}), 25.4 (C₄), 22.5 (C₂), 14.0 (C₆) ppm. Purity: 98% (by HPLC).

**Example 1.8. Synthesis of *n*-hexyl 4-[2-(1-benzyl-piperidin-4-yl)-ethylcarbamoyl]-2-[(thiophen-2-carbonyl)-amino]-butyrate.** Yellow solid of melting point: 48-50 ºC. MS (ESI⁺): *m*/*z* = 542 [M+H]⁺. ¹H-NMR (400 MHz, MeOD) δ: 7.85 (dd, 1H, *J* = 3.9, 1.2 Hz, H_{3"}), 7.67 (dd, 1H, *J* = 5.1, 1.2 Hz, H_{5"}), 7.43 (s, 5H, H_{10'}-H_{14'}), 7.13 (dd, *J* = 5.1, 3.9 Hz, H_{4"}), 4.50 (m, 1H, H_{α}), 4.14 (m, 2H, H₁), 4.13 (s, 2H, H_{8'}), 3.34-3.29 (m, 3H, H_{6'eq}, H_{5'eq}, H_{3'}), 3.16 (m, H, H_{1'}), 2.74 (m, 2H, H_{6'ax}, H_{5'ax}), 2.35 (m, 2H, H_{γ}), 2.28 (m, 1H, H_{β}), 2.06 (m, 1H, H_{β}) 1.87 (m, 2H, H_{7'eq}, H_{4'eq}), 1.64 (m, 2H, H₂), 1.42 (m, 2H, H_{2'}), 1.34-1.26 (m, 8H, H_{7'ax}, H_{4'ax}, H₃, H₄, H₅), 0.86 (t, 3H, *J* = 7.0 Hz, H₆) ppm.¹³C-NMR (100 MHz, MeOD) δ: 174.6 (*C*OC_{γ}), 173.3 (*C*OC_{α}), 164.5 (*C*ONHC_{α}), 139.6 (C_{2"}, C_{9'}), 132.2 (C_{3"}), 132.0 (C_{10'},C_{14'}), 130.5 (C_{5"}), 130.2 (C_{12'}), 130.1 (C_{11'}, C_{13'}), 128.9 (C_{4"}), 66.5 (C₁), 62.2 (C_{8'}), 54.0 (C_{α}, C_{3'}), 53.7 (C_{5'},C_{6'}), 37.4 (C_{1'}), 36.2 (C_{2'}), 33.2 (C_{γ}), 32.5 (C_{4'}, C_{7'}), 30.7 (C₃), 29.6 (C₂), 27.8 (C_{β}), 26.7 (C₄), 23.6 (C₅), 14.3 (C₆) ppm. Purity: 96% (by HPLC).

**Example 1.9. Synthesis of *n*-hexyl 4-[2-(1-benzyl-piperidin-4-yl)-ethylcarbamoyl]-2-[(4-bromo-thiophen-2-carbonyl)-amino]-butyrate.** Yellow oil. MS (ESI⁺): *m*/*z* = 622, 620 [M+H]⁺. ¹H-NMR (400 MHz, MeOD) δ: 7.74 (d, 1H, *J* = 1.4 Hz, H_{5"}), 7.68 (d, *J* = 1.4 Hz, H_{3"}), 7.36 (s, 5H, H_{10'}-H_{14'}), 4.48 ( m, 1H, H_{α}), 4.14 (m, 2H, H₁), 3.73 (s, 2H, H_{8'}), 3.30 (m, 2H, H_{6'eq}, H_{5'eq},), 3.13 (m, 2H, H_{1'}), 2.40-2.36 (m, 3H, H_{6'ax}, H_{5'ax}, H_{3'}), 2.32 (m, 1H, H_{β}), 2.07 (m, 1H, H_{β}), 1.77-1.74 (m, 2H, H_{7'eq}, H_{4'eq}), 1.40 (m, 2H, H₂), 1.32 (m, 2H, H_{2'}), 1.31-1.27 (m, 8H, H_{7'ax}, H_{4'ax}, H₃, H₄, H₅), 0.87 (t, 3H, *J* = 7.02 Hz, H₆) ppm. ¹³C-NMR (100 MHz, MeOD) δ: 174.6 (*C*OC_{γ}), 173.1 (*C*OC_{α}), 164.5 (*C*ONHC_{α}), 141.0 (C_{2"}, C_{9'}), 132.2 (C_{3"}), 131.4 (C_{10'},C_{14'}), 129.9 (C_{5"}), 129.6 (C_{12'}), 129.3 (C_{11'}, C_{13'}), 111.0 (C_{4"}), 66.6 (C₁), 63.5 (C_{8'}), 54.2, (C_{α}), 54.1 (C_{3'}), 53.8 (C_{5'},C_{6'}), 37.7 (C_{1'}), 36.6 (C_{2'}), 33.5 (C_{γ}), 32.5 (C_{4'}, C_{7'}), 31.8 (C₃), 29.6 (C₂), 27.8 (C_{β}), 26.7 (C₄), 23.6 (C₅), 14.3 (C₆) ppm. Purity: 97% (by HPLC).

### Example 2. Neuroprotective properties of the compounds of the present invention

The primary failures that cause AD in particular and many other ND in general are not completely understood. However, there exists a large amount of evidence that phenomena of an oxidative type are involved in this etiology, particularly the oxidative stress produced by oxygen radicalic species generated in an uncontrollable amount. Such species can be of external origin (photooxidation, emissions, etc.) as well as of internal origin (mitochondrial metabolism, neutrophilic activation, etc.).

From the chemical point of view these species can be free radicals of the type superoxide or hydroxyl, non-radical oxygen like hydrogen peroxide or peroxynitrite or reactive molecules like cetaldehydes or hydroxynonenal (Mutat. Res. 1999, 428, 17-22). The healthy brain possesses mechanisms capable of eliminating these oxidative species, transforming them into others that are innocuous. But in pathological situations, or simply under risk factors such as ageing, these species can escape to the control systems and lead to aberrant biochemical processes (J. Neurosci. 2001, 21, 4183-4187). This indicates that molecules capable of replacing or complementing those brain mechanisms responsible for maintaining the oxygen reactive species at acceptable levels would exert a neuroprotective effect by eliminating or reducing such radicalic species and the damage that they would cause.

With this goal in mind, the cytoprotective effect of the compounds was evaluated in human neuroblastoma cells, specifically the neuroprotective potential of these compounds against oxidative stress produced in two different models: with hydrogen peroxide as exogenous generator of free radicals, and with rotenone and oligomycin A, blockers of the mitochondrial respiratory chain, as described in J. Neurochem. 1992, 59, 1609-1623 and in Toxicological Sciences 2004, 79, 137-146, respectively. In both models, the catalytic activity of the enzyme lactate dehydrogenase (LDH), an enzyme which is released to the extracellular medium after cell death (J. Pharmacol. Exp. Ther. 2005, 315, 1346-1353), was used as a parameter of cell viability.

Following a procedure previously described (Neuropharmacology 2004, 46, 103-114), the following experimental conditions were used: cells from human neuroblastoma cell line SH-SY5Y were plated and cultured in a medium DMEM (Dulbecco's modified Eagle's médium) containing 15 non-essential amino acids and supplemented with 10% fetal calf serum, glutamine 1 milimolar, 50 units per milliliter of penicillin and 50 micrograms per milliliter of streptomycin, and kept at 37ºC in humidified air containing 5% carbon dioxide. In the assays, SH-SY5Y were sub-cultured in 48-well plates with a density of seed of 5x10⁵ cells per well, or in 96-well plates with a density of 2x10⁵ cells per well. In the cytotoxicity experiments, cells prepared in this way were treated with the compounds to test, in DMEM free of serum.

To study the cytoprotective action of different compounds against cell death induced by hydrogen peroxide 30 micromolar or by a combination of rotenone 30 micromolar with oligomycin A 10 micromolar, compounds were added to the medium and maintained there for 24 hours. Then, media were replaced by fresh media still containing the compound plus the cytotoxic agent, and were maintained there for an additional period of 24 hours. Cell viability was checked by measuring the activity of the enzyme LDH, as it was explained before, using the kit "Cytotoxicity Cell Death" (Roche-Boehringer Mannheim) following the instructions of the manufacturer. Total LDH activity was defined as the sum of intra plus extra LDH activities. Released LDH was defined as the percentage of extracellular compared with total LDH activity.

In all the pharmacological experiments a positive control was used as comparison and to evaluate the reliability of the method used. In this case trolox, a well known free radical scavenger that is the active nucleus of the natural antioxidant vitamin E (New. Engl. J. Med. 2005, 352, 2379-2388), was used. The results obtained for the compounds described as Examples 1.1 to 1.9 that are shown here, are expressed in percentage of the neuroprotective activity.

**2.1 Neuroprotection against a toxic stimulus of oxygen peroxide (60 micromolar),** referred to the percentage of protection induced by each compound, at the concentration expressed that caused the maximum protection in each case:

| Compd. | Concentration (micromolar) | Protection (%) |
|---|---|---|
| **Example 1.1** | 10 | 39.1 |
| **Example 1.2** | 1 | 24.9 |
| **Example 1.3** | 3 | 68.1 |
| **Example 1.4** | 1 | 28.7 |
| **Example 1.5** | 3 | 32.5 |
| **Example 1.6** | 30 | 48.8 |
| **Example 1.7** | 10 | 46.4 |
| **Example 1.8** | 10 | 73.0 |
| **Example 1.9** | 3 | 84.3 |
| **Trolox** | 30 | 57.7 |

**1.2 Neuroprotection against a toxic stimulus of a combination of rotenone (30 micromolar) plus oligomycin A (10 micromolar),** referred to the percentage of protection induced by each one of the compounds selected for this test, at the concentration expressed that caused the maximum protection in each case:

| Compd. | Concentration (micromolar) | Protection (%) |
|---|---|---|
| **Example 1.3** | 0.3 | 47.9 |
| **Example 1.7** | 0.3 | 46.1 |
| **Example 1.8** | 0.1 | 48.9 |
| **Example 1.9** | 3 | 35.9 |
| **Trolox** | 3 | 52.9 |

These results indicate that the compounds of the present invention are able to reduce the presence of exogenous or endogenous radicalic species, which make them potential medicines for the treatment of pathologies generated or favored by oxidative stress or by the presence of radicalic species in general.

### Example 3. Properties of the compounds of the present invention as inhibitors of the enzyme acetylcholinesterase

AD, like all ND, is a very slow process with an average time of 8 years between the first symptoms and the death. The first symptoms appear as short term memory loss, with the not recognition of relatives or the forgetting of normal skills, language problems, cognitive alterations, loss of learning capacity and difficulties in orientation, which increase until the total loss of the cognitive capacities while the illness progresses (Ann. Intern. Med. 2004, 140, 501-509).

The immediate cause of these cognitive failures, at least in the initial phases, is the reduction in the levels of neurotransmitters involved in the synaptic communication. The clinical symptoms of AD are related with the loss of cholinergic neurons in the nucleus basal of Meynert, the cholinergic synapses which utilize ACh as neurotransmitter being the most affected in this illness. Taking into account the devastating effects of the illness, one understands the importance of the symptomatic treatments, since they are capable of replacing, although temporarily and with limited efficacy, the cognitive abilities of AD patients. For this reason AChE inhibitors, which prevent the degradation of ACh and increase its synaptic levels, have been the only drugs used during the last years in the treatment of AD, and even now they continue to be almost the only drugs in the market for the treatment of this illness.

For this reason, it was considered interesting to evaluate the capacity to inhibit AChE of the compounds of the present invention. This evaluation was performed following the method of Rappaport (Clin. Chim. Acta 1959, 4, 227), using purified AChE from *Electrophorus electricus* and acetylcholine chloride (29.5 milimolar) as substrate. The reaction took place in a final volumen of 2.5 milliliters of an aqueous solution containing 0.78 units of AChE and m-nitrophenol at 1.9 milimolar to produce a yellow color, which is lost in function of the enzyme activity.

Curves of inhibition were made incubating with the compounds of the present invention for 30 minutes and a sample without any compound was prepared to determine the 100% of the enzymatic activity. After 30 minutes of incubation, the loss of yellow color was evaluated by measuring absorbance at 405 nanometers in a spectrophotometric microplate reader (iEMS Reader MF, Labsystems). The concentration of compound producing 50% inhibition of AChE activity (IC₅₀) was calculated transforming the values of absorbance to enzymatic activity Rappaport units extrapolating from a calibration curve previously obtained.

The results of inhibition of AChE, expressed as the concentration which inhibits 50% of activity (IC₅₀) for the compounds of the present invention, are the following:

| Compd. | AChE-I, (micromolar) | IC₅₀ |
|---|---|---|
| **Example 1.1** | 1.4 | |
| **Example 1.2** | 5 | |
| **Example 1.3** | 1.9 | |
| **Example 1.4** | 2.5 | |
| **Example 1.5** | 2.3 | |
| **Example 1.6** | 3.25 | |
| **Example 1.7** | 3.5 | |
| **Example 1.8** | 3.5 | |
| **Example 1.9** | 1.2 | |

These results show that the family of compounds of the present invention can be also medicines useful for the symptomatic treatment of AD.

### Example 4. Properties as propidium iodide displacers from the peripheral site of the enzyme acetylcholinesterase of the compounds of the present invention

Numerous ND have in common conformational changes of certain proteins that finally lead to its aberrant polymerization and accumulation. AD shares this characteristic, since the amyloid peptide soluble and physiologically normal changes its conformation and forms soluble lineal small oligomers which grow to form fibrils and finally precipitates in extracellular aggregates of big size call senile plaques. Perhaps because they are histological apparent, the amyloid plaques used to occupy the center of the original amyloid hypothesis but today it is given more importance to the neurotoxic capacity of intermediate pre-fibrillar forms, soluble oligomers and protofibrills produced during the formation of plaques (J. Neurochem. 2007, 101, 1172-1184), and results evident that polymerization and aggregation of monomers of the amyloid peptide are becoming important therapeutic targets.

Several studies have demonstrated that the acetylcholinesterase enzyme binds to this beta amyloid peptide, inducing the formation of fibrils (Neurochem. Res. 1998, 23, 135) and that the peripheral anionic site of the enzyme is involved in this adhesion (Biochem. Pharmacol. 2003, 65, 407-416).

It is known that some ligands such as propidium iodide, which binds to the peripheral anionic site of the enzyme, inhibit the aggregation of beta amyloid (Mol. Psychiatry 1996, 1, 359). Therefore, it was considered of interest to know whether any of the compounds of the invention were able to bind to the peripheral site, which would indicate a possible anti-aggregate effect of beta amyloid.

A competition test for the peripheral site of acetylcholinesterase was carried out with the compounds at concentrations of 0.3, 1 and 3 micromolar. Propidium, a specific ligand of the peripheral site, causes an increase in fluorescence on joining this site (Mol. Pharmacol. 1987, 31, 610). A reduction in fluorescence due to propidium in the presence of the compounds could be interpreted as a displacement of propidium from the peripheral site.

A solution of AChE from bovine erythrocytes at the concentration of 5 micromolar in Tris buffer 0.1 milimolar and pH 8 was used. Aliquots of the compounds of the present invention were added, Examples 1.1 to 1.9, at a final concentration of 0.3 micromolar, and these solutions were maintained at room temperature for at least 6 hours. After that period of time, samples were incubated for 15 minutes with propidium at a final concentration of 20 micromolar and fluorescence was measured in a fluorescence microplate reader (Fluostar Optima, BMG, Germany) at the excitation and emission waves of 485 nm and 620 nm respectively.

The results obtained (which will be explained below) are expressed as the percentage of the reduction in fluorescence induced by each compound at the indicated concentration, which is that producing the maximum reduction in each case As a positive control for the comparison and evaluation of the suitability of the method employed, the compound BW284c51 (Sigma-Aldrich, Spain) was utilized at a concentration of 1 milimolar.

| Compd. | Concentration (micromolar) | Protection (%) |
|---|---|---|
| **Example 1.1** | 3 | 29.3 |
| **Example 1.2** | 1 | 43.6 |
| **Example 1.3** | 3 | 37.3 |
| **Example 1.4** | 3 | 39.7 |
| **Example 1.5** | 3 | 23.4 |
| **Example 1.6** | 0.3 | 37.4 |
| **Example 1.7** | 0.3 | 42.2 |
| **Example 1.8** | 3 | 15.0 |
| **Example 1.9** | 0.3 | 11.3 |
| **BW284c51** | 1000 | 24.5 |

The data obtained show that the compounds of the present invention are able to bind at the peripheral site of the AChE enzyme, which makes them potential drugs with an anti-aggregate effect on the beta amyloid peptide.

### Example 5. Capacity for penetration in the central nervous system

Taking into account the central role that the brain plays in the organism, it should be expected that Nature has protected it in a special way. In fact, the whole CNS, but particularly the brain, appears as an armoured organ, a separate system within the organism as a whole, separated by a membrane of special characteristics called the blood brain barrier (BBB) which selects the passage of certain molecules in both directions. This means that it is useless to obtain a product exhibiting a high activity *in vitro,* with great potential for the treatment of, for example, ND if *in vivo* it is not able to reach its therapeutic target. It then becomes of great importance to know *a priori* if a molecule or a family of molecules that are the object of an investigation of the type that is presented in this patent, are capable of crossing the BBB before reaching later phases of drug development, in order to introduce new modifications in the molecule, or even stop the line of investigation if it is confirmed that the products are not able of penetrating the CNS through the BBB. In both cases, this experiment gets high cost savings.

Among the different methods to evaluate the *in vitro* CNS penetration capacity, the methodology PAMPA-BBB (Parallel Artificial Membrane Permeation Assay for Blood-Brain Barrier, described in Eur. J. Med. Chem. 2003, 38, 223-232) is the most used due to the high precision and reliability. In the case of products of this invention, we chose three representative compounds of this family to study their ability to cross the BBB using PAMPA methodology, in the manner described below.

The experiments were performed using two 96-well microplate in a sandwich assembly. The top microplate (Millipore, Ref. MAIPS4510) is provided with 96 hydrophobic PVDF filters, where a solution of a lipid extract of pig brain (Avanti Polar Lipids) in dodecane is put down.

The bottom microplate has 96 tear-shaped wells (Millipore, Ref. MAMCS9610). The acceptor 96-well microplate was filled with 180 µL per well of a mixture of phosphate buffer saline pH 7.4 (PBS) and EtOH in proportion 90:10. The filter surface of the donor microplate was impregnated with 4 µL of a solution of lipid extract from pig brain in dodecane (20 mg mL⁻¹).

Compounds were dissolved in PBS / EtOH (90:10) and then 180 µL were added to the donor microplate, which is carefully placed on the receiver plate. After 120 minutes of incubation at 25 °C, plate donor is carefully separated and determined the concentration of the compounds in the acceptor plate by UV spectroscopy. This method measures the permeability, expressed as the rate of passage of a given barrier in 10⁻⁶ centimeter per second (10⁻⁶ cm s⁻¹). The results are expressed as the mean plus / minus the standard deviation of three independent assays, each containing four replicates of each tested compound. Previously, the method was validated with 15 commercial drugs: testosterone, verapamil, imipramine, desipramine, progesterone, promazine, chlorpromazine, clonidine, piroxicam, caffeine, aldosterone, lomefloxacin, enoxacin, atenolol and ofloxacin, which provided values between 9.7 ± 0.1 (testosterone) and 0.7 ± 0.01 (ofloxacin), consistent with the permeability values reported for these compounds.

The results were:

| Comp. | Permeability^{a} | Classification |
|---|---|---|
| **Example 1.2** | 4.4 ± 0.1 | CNS + |
| **Example 1.6** | 3.2 ± 0.01 | CNS + |
| **Example 1.8** | 5.3 ± 0.01 | CNS + |

| | | |
|---|---|---|
| ^{a}The experimental permeability, *P*ₑ (10⁻⁶ cm s⁻¹), is the mean ± SD of three independent assays containing four replicates of each tested compound, as described in Eur. J. Med. Chem. 2003, 38, 223-232. ^{b}CNS + means that compound penetrates into the CNS and CNS - means that compound does not penetrate into the CNS. | | |

In conclusion, the products covered by this invention are able to cross the BBB, to reach their therapeutic targets in the brain, an essential condition for potential drugs for the treatment of diseases of the nervous system, such as ND including AD.

## Claims

1. A compound of general formula (I) 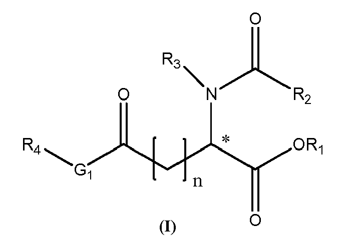 wherein,
R₁ represents a hydrogen atom or an aryl, heteroaryl, alkyl, haloalkyl, aminoalkyl, ammoniumalkyl group and, in general, straight or branched alkyl groups, substituted for any chemical group with the express exclusion of alkyl groups substituted by aryl, such as a benzyl group.
R₂, R₃ are independently selected from hydrogen, substituted or unsubstituted aryl, heteroaryl, alkyl, carbamate, in which R₂ represents -O-aryl or-O-alkyl groups, straight or branched, unsubstituted or substituted for any chemical group, urea derivatives in which R₂ represents -NH-aryl or -NH-alkyl groups, straight or branched, unsubstituted or substituted by any chemical group, imides. In general, any chemical group capable of generating the molecule represented in the general formula (I).
n is 1 or 2.
C* represents the chiral carbon in amino acids, and may be the R enantiomer, the S enantiomer, or a mixture in any proportion of both enantiomers.
G₁ represents O, N mono or disubstituted, CH₂, S or, more generally, any group or chemical structure that can be used as a bond between the amino acid and R₄.
R₄ represents any chemical structure combining substituted or unsubstituted alkyl, aryl or heteroaryl groups.
or an isomer, a pharmaceutically acceptable salt and / or a solvate thereof.

2. A compound according to claim 1 wherein R₁ is a C₂-C₈ alkyl group, straight or branched.

3. A compound according to claim 2 wherein R₁ is a C₅-C₇ straight alkyl group.

4. A compound according to claim 1 in which R₃ is a hydrogen atom or a C₁-C₃ alkyl group.

5. A compound according to claim 1 in which G₁ is an amino group optionally substituted with one or two C₁-C₃ alkyl groups.

6. A compound according to claim 1 where n is 2.

7. A compound according to claim 1 in which R₄ represents a piperidine-4-yl-ethyl group, optionally substituted.

8. A compound according to claim 7 wherein the piperidine-4-yl-ethyl group is substituted in its nitrogen atom with an arylalkyl group.

9. A compound according to claim 8 wherein the arylalkyl group is a benzyl group, optionally substituted with 1-3 groups selected from C₁-C₃ alkyl, C₁-C₃ alkoxy, cyano, nitro, and halogen atoms.

10. A compound according to claim 9 in which the arylalkyl group is a non-substituted benzyl group.

11. A compound according to claim 1, selected from the group consisting of:
• *n*-Hexyl 2-benzyloxycarbonylamino-4-[2-(1-benzylpiperidin-4-yl)-ethylcarbamoyl]-butyrate.
• *n*-Hexyl 4-[2-(1-benzylpiperidin-4-yl)-ethylcarbamoyl]-2-tert butoxycarbonylamino-butyrate.
• *n*-Hexyl 2-benzoylamino-4-[2-(1-benzylpiperidin-4-yl)-ethylcarbamoyl]-butyrate.
• *n*-Hexyl 2-[(benzo[*b*]tiofen-2-carbonyl)-amino]-4-[2-(1-benzyl-piperidin-4-yl)-ethylcarbamoyl]-butyrate.
• *n*-Hexyl 4-[2-(1-benzyl-piperidin-4-yl)-ethylcarbamoyl]-2-[2-(6-chloro-benzo[*b*]thiophen-2-yl)-acetylamino]-butyrate.
• *n*-Hexyl 4-[2-(1-benzyl-piperidin-4-yl)-ethylcarbamoyl]-2-[(thieno[*2*,*3-b*]piridin-2-carbonyl)-amino]-butyrate.
• *n*-Hexyl 4-[2-(1-benzyl-piperidin-4-yl)-ethylcarbamoyl]-2-[(thieno[*2*,*3-b*]thiophen-2-carbonyl)-amino]-butyrate.
• *n*-Hexyl 4-[2-(1-benzyl-piperidin-4-yl)-ethylcarbamoyl]-2-[(thiophen-2-carbonyl)-amino]-butyrate.
• *n*-Hexyl 4-[2-(1-benzyl-piperidin-4-yl)-ethylcarbamoyl]-2-[(4-bromo-thiophen-2-carbonyl)-amino]-butyrate.

12. A pharmaceutical composition which comprises a compound of formula (I) according to any of claims 1 to 11, together with one or more pharmaceutically acceptable carrier, adjuvant or vehicle.

13. A composition according to claim 12 which comprises one or more additional therapeutic agents.

14. Use of a compound of general formula (I) 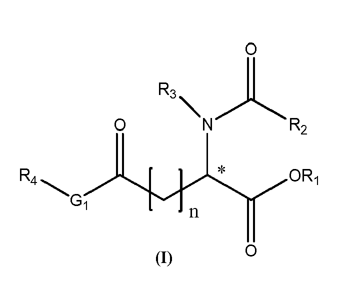 wherein R₁, R₂, R₃, R₄, G₁ and n have the meanings defined in any of claims 1-11 or an isomer, a pharmaceutically acceptable salt and / or a solvate thereof for the manufacture of a medicament for the prevention and / or treatment of disorders or diseases in which oxidative processes are implicated as the etiology of these disorders or diseases.

15. Use according to claim 14 wherein the conditions or diseases are those in which a deficit or dysfunction of cholinergic neurotransmission is involved.

16. Use according to claim 14 wherein the conditions or diseases are those in which the aggregation of amyloid peptide is involved in different degrees: oligomers, protofibrils, fibrils, fibrillar aggregates, amyloid plaques, etc.

17. Use according to any of claims 14-16 wherein the disorder or disease belongs to a group of ND.

18. Use according to claim 17 wherein the ND is AD, Parkinson's disease, Huntington's disease or any other **characterized by** neurodegenerative processes such as neuronal loss, failure of neurotransmission processes or appearance of amyloid peptide aggregates.

19. Procedures for obtaining a compound of general formula (I) 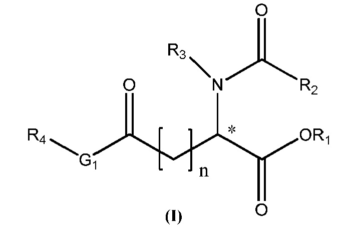 wherein,
R₁ represents a hydrogen atom or an aryl, heteroaryl, alkyl, haloalkyl, aminoalkyl, ammoniumalkyl group and, in general, straight or branched alkyl groups, substituted for any chemical group with the express exclusion of alkyl groups substituted by aryl, such as a benzyl group.
R₂, R₃ are independently selected from hydrogen, substituted or unsubstituted aryl, heteroaryl, alkyl, carbamate, in which R₂ represents -O-aryl or-O-alkyl groups, straight or branched, unsubstituted or substituted for any chemical group, urea derivatives in which R₂ represents -NH-aryl or -NH-alkyl groups, straight or branched, unsubstituted or substituted by any chemical group, imides. In general, any chemical group capable of generating the molecule represented in the general formula (I).
n is 1 or 2.
C* represents the chiral carbon in amino acids, and may be the R enantiomer, the S enantiomer, or a mixture in any proportion of both enantiomers.
G₁ represents O, N mono or disubstituted, CH₂, S or, more generally, any group or chemical structure that can be used as a bond between the amino acid and R₄.
R₄ represents any chemical structure combining substituted or unsubstituted alkyl, aryl or heteroaryl groups.
or an isomer, a pharmaceutically acceptable salt and / or a solvate thereof, **characterized by** the following steps:
a) Reaction of a product of formula (II) 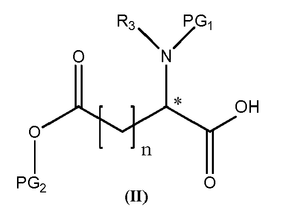 wherein PG₁ is a protecting group of hydroxyl groups and PG₂ is a protecting group of amino groups, with an alcohol of formula R₁-OH in an anhydrous solvent to obtain compounds of formula (III). 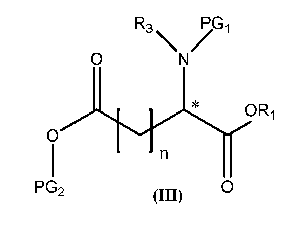
b) Deprotection of the hydroxyl group in the compounds of formula (III) by removing the protecting group of hydroxyl groups PG₂, to give the compound of formula (IV) 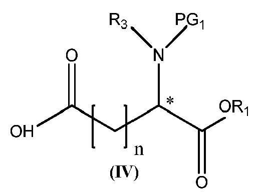
c) Reaction of a compound of formula (IV) with a compound of formula H-G₁-R₄ in the presence of a coupling agent to give compounds of formula (Ia). 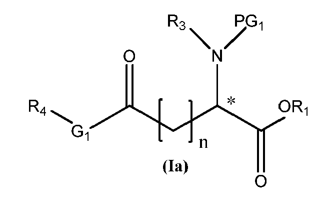 Optionally, the hydrolysis of compound (Ia) in which R₂ is a *tert-*butyloxycarbonyl group by treatment with a strong acid and subsequent acylation of the resulting product by reaction with an acyl chloride of formula R₂COCl or an anhydride of formula (R₂CO)₂O in an anhydrous non-polar solvent.
